# FASCICULE DE BREVET EUROPEEN

(11) **EP 3 362 078 B1**
(45) Date de publication et mention de la délivrance du brevet: **04.12.2019**
(21) Numéro de dépôt: 16794376.0
(22) Date de dépôt: 13.10.2016
(51) Int. Cl.: A61K 36/00, B01D 11/02, A23L 33/105

(54) **NOUVEAU SOLVANT D'EXTRACTION ET/OU DE SOLUBILISATION ORGANIQUE, PROCÉDÉ D'EXTRACTION METTANT EN OEUVRE LEDIT SOLVANT, ET EXTRAITS ISSUS DUDIT PROCÉDÉ**
NEUARTIGES ORGANISCHES AUFLÖSUNGS- UND/ODER EXTRAKTIONSLÖSUNGSMITTEL, EXTRAKTIONSVERFAHREN MIT VERWENDUNG DES BESAGTEN LÖSUNGSMITTELS UND DURCH BESAGTES VERFAHREN HERGESTELLTE EXTRAKTE
NOVEL ORGANIC SOLUBILISATION AND/OR EXTRACTION SOLVENT, EXTRACTION METHOD USING SAID SOLVENT, AND EXTRACTS OBTAINED BY SAID METHOD

(30) Priorité: 14.10.2015 FR 1559749
(43) Date de publication de la demande: 22.08.2018
(73) Titulaire: Naturex (Société Anonyme), 84911 Avignon (FR); Université d'Avignon et des Pays de Vaucluse (Etablissement Public), 84000 Avignon (FR)
(72) Inventeur: ROMBAUT, Natacha, 54000 Nancy (FR); FABIANO TIXIER, Anne-Sylvie, 30650 Rochefort du Gard (FR); BIRTIC, Simona, 84300 Cavaillon (FR); BILY, Antoine, Charles, 84270 Vedene (FR); ROLLER, Marc, 84310 Morieres les Avignon (FR); CHEMAT, Farid, 84310 Morieres les Avignon (FR)
(74) Mandataire: Potter Clarkson
(86) Numéro de dépôt international: PCT/FR2016/052643
(87) Numéro de publication internationale: WO 2017/064424

(56) Documents cités:
- WO-A1-2012/012856
- FR-A1- 2 892 933
- US-A- 5 938 859
- US-A1- 2004 102 351
- US-A1- 2014 346 391

## Description

### [INTRODUCTION]

La présente invention concerne un nouveau solvant d'extraction et/ou de solubilisation organique comprenant au moins 99 % de para-menthane, un procédé d'extraction mettant en œuvre ledit solvant, et les extraits issus dudit procédé.

Plus particulièrement, l'invention concerne une nouvelle utilisation du para-menthane, composé terpénoïde monocyclique saturé, en tant que solvant dérivé de substances naturelles, et vise également un solvant comprenant du para-menthane.

L'invention a également pour but de définir un procédé d'extraction et de solubilisation mettant en œuvre ledit solvant notamment dans des procédés d'extraction liquide / liquide ou solide / liquide, ainsi que les extraits issus de la mise en œuvre de ce procédé.

Le solvant selon l'invention peut être utilisé pour l'extraction et/ou la solubilisation de matériel biologique végétal et/ou animal et/ou procaryote.

Préférentiellement, ledit solvant sera mis en œuvre pour l'extraction et la solubilisation de composés naturels actifs à partir d'un extrait végétal ou de matières végétales d'origine naturelle.

Les composés actifs naturels sont des molécules issues d'une matière végétale ayant une activité d'intérêt biologique et technologique. Ces composés actifs naturels peuvent être sous forme pure ou être contenus dans des extraits. Les composés actifs naturels font partie de la catégorie des pigments, comprenant les caroténoïdes et les chlorophylles, des arômes comprenant les terpènes, des huiles végétales comprenant les triglycérides ou les phospholipides. Ces composés actifs peuvent être utilisés dans des applications alimentaires, cosmétiques, pharmaceutiques et nutraceutiques.

FR 2,892,933 concerne un extrait végétal obtenu par un procédé d'extraction en une seule phase, à l'aide d'un solvant et/ou d'un mélange de solvants d'origine végétale dont les constituants sont caractérisés et chimiquement définis, caractérisé en ce qu'il est exempt de polyphénol, d'anthocyane et/ou de tanin et qu'il comporte une fraction de composés à caractère lipophile et/ou une fraction de composés à caractère polaire. Elle concerne également un procédé permettant d'obtenir un extrait selon l'invention.

US 5,938,859 concerne un mélange de solvants comprenant du bromure de n-propyle, un mélange de solvants à bas point d'ébullition et, de préférence, un additif de défluxage et / ou d'élimination ionique et / ou au moins un terpène saturé.

### [ETAT DE L'ART ANTERIEUR]

Le terme « composés naturels » fait référence à différentes catégories de composés biochimiques. Mis a part les corps gras végétaux, ces composés sont habituellement présents en faible quantité dans les matrices végétales. On distingue ainsi plusieurs catégories de composés naturels selon leur nature chimique :
- Les pigments : peuvent être présents sous la forme de chaines carbonées partiellement hydroxylées et contenant un cycle aromatique. Les pigments lipophiles peuvent être obtenus par concentration d'extraits obtenus des végétaux appelés oléorésines. Les pigments sont différenciés en fonction de leur couleur, par exemple : les caroténoïdes (couleur jaune, orange ou rouge), les anthocyanes (couleur rouge à violette voire bleue), les xanthophylles et curcuminoides (couleur jaune), les chlorophylles (couleur verte) et la phycocyanine (couleur bleue).
- les arômes : cette catégorie de composés fait référence à des composés volatils présents dans les huiles essentielles, les concrètes et absolues de fleurs par exemple. Les molécules de type terpéniques (leur structure chimique est composée d'un cycle aromatique contenant des groupements hydroxyles) représentent une grande partie des arômes. Les profils aromatiques des plantes sont dominés par quelques terpènes en fonction des végétaux. Par exemple, l'huile essentielle de basilic est principalement composée de linalol, d'estragol, d'eugénol et de méthyl eugénol.
- les huiles végétales : ces mixtures contiennent des composés ayant pour structure chimique principale une chaîne carbonée allant classiquement de 14 à 20 atomes de carbone (les unités composant les corps gras sont appelées acides gras) et une fraction insaponifiable (lipides polaires, stérols etc..). Les huiles végétales sont des composés lipophiles et les extraits contenant des corps gras sont sous la forme d'huile ou de matière végétale grasse solide contenant des triglycérides ou phospholipides.

Pour l'obtention de composés naturels, la mise en œuvre la plus pratiquée est l'extraction solide / liquide. Il existe plusieurs procédés usuels d'extraction solide / liquide, tels que la macération, la percolation, la lixiviation, décoction. Traditionnellement, ces procédés mettent en œuvre l'eau et l'éthanol pour l'obtention d'extraits naturels. Cependant ces solvants permettent de solubiliser principalement des composés hydrophiles avec une faible sélectivité.

D'autres solvants sont utilisés pour une extraction plus sélective. Les solvants les plus utilisés en extraction de substances naturelles sont des solvants organiques tels que l'hexane, le méthanol, le dichlorométhane et l'acétone. Ces solvants sont des substances très efficaces qui dissolvent les solutés solides et liquides, et leur volatilité facilite leur élimination en fin de Procédé. Cependant, les solvants organiques, sont aussi, pour bon nombre d'entre eux, inflammables, très volatils et toxiques (cancérigènes, mutagènes, etc.). Plusieurs travaux ont déjà été réalisés sur leurs effets nocifs (Baldasseroni et al., 2003; Manini et al., 2004). Par ailleurs, l'épuisement progressif des ressources pétrolières et les contraintes réglementaires croissantes incitent à s'orienter vers des solvants alternatifs. Ainsi, de nombreuses études visent plus particulièrement le remplacement de l'hexane par d'autres solvants qui permettraient d'obtenir un rendement d'extraction équivalent et qui seraient moins nocifs

L'hexane est en effet une molécule classée dans la catégorie des molécules de type « Cancérogène, Mutagène et Reprotoxique » (CMR) de classe 3 (liste CMR UB) soit comme molécule reprotoxique. Les caractéristiques physico-chimiques de l'hexane rendent sa manipulation dangereuse. L'hexane possède un point éclair bas (23,3°C) et une température d'auto-inflammation de 233,9°C.

Pour remplacer l'hexane, différents solvants ont été proposés.

Les huiles végétales peuvent être utilisées en tant que solvant d'extraction de composés non volatils. L'enrichissement des huiles est alors limité par la solubilité des extraits végétaux dans l'huile, ne permettant qu'une extraction partielle des composés naturels contenus dans les végétaux traités. Les agro-solvants de type méthyl-THF ont été utilisés pour l'extraction (Sicaire et al., 2014) cependant la principale contrainte industrielle repose sur la réactivité du 2-méthyl-THF avec les matériaux plastiques.

D'autres agro-solvants de type terpéniques obtenus à partir de co-produits de l'industrie agro-alimentaire ont été proposés pour l'extraction de composés naturels. Les solvants terpéniques tels que le limonène ou l'alpha-pinène (par exemple WO 2007/057349 A2) peuvent avoir une réactivité chimique due à la présence de doubles liaisons. Thomas et Bessière (1989) indiquent qu'il existe de nombreuses transformations du limonène suite à des réactions chimiques par exemple de type acido-basiques, epoxydations, hydrogénations et oxydations. Le limonène constitue donc une espèce réactive en solution. Les transformations du limonène impliquent une modification de sa structure par réarrangement et réaction des doubles liaisons avec d'autres espèces chimiques présentes en solution.

On cannait de Karlberg et al., (1992) que le limonène exposé à l'air subit des réactions d'auto-oxydation conduisant à la formation de dérivés oxygénés de type hydroperoxydes. Ces composés formés seraient de plus allergisants. De plus, la réaction d'auto-oxydation des terpenoides (tels que le limonène, le thymol, le cytral et l'alpha-pinène par exemple) engendre la production de radicaux libres (Turek et Stintzing 2013). Ceux-ci peuvent réagir avec les composés cibles d'extraction conduisant à leur dégradation.

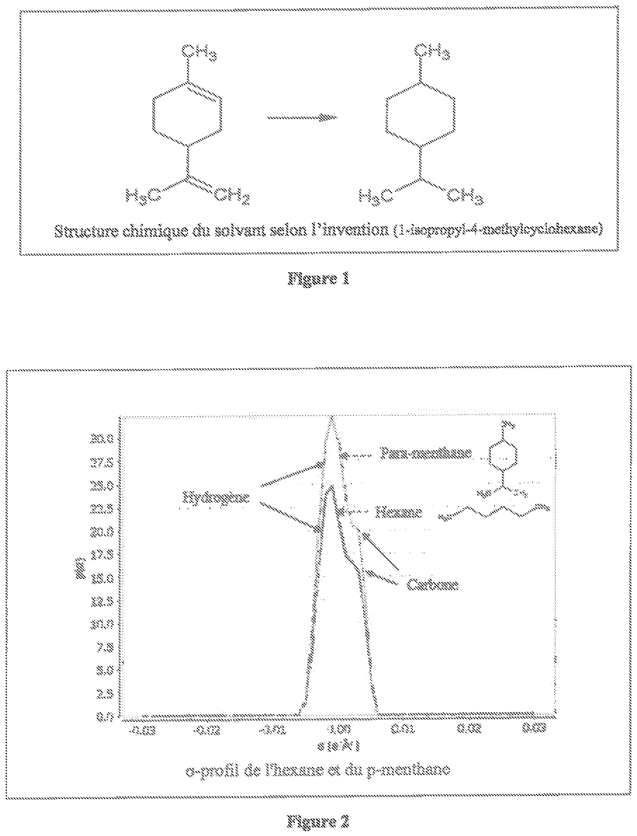

Pour pallier aux inconvénients des composés terpéniques contenant des doubles liaisons, il serait possible de considérer un analogue terpénique qui ne contiendrait pas de doubles liaisons. Plusieurs équipes de chimistes mentionnent l'obtention d'une molécule nommée para-menthane (1-isopropyl-4-methylcyclohexane, figure 1) par hydrogénation catalytique (brevet US 2013/0281747 A1 de Chirik et al., 2013, Li *et al.,* 2014, Shuikin and Cherkashin, 1957).

Le para-menthane a fait l'objet de plusieurs mentions dans différents brevets.

Le para-menthane a été mentionné pour des applications chimiques, notamment en tant que composé formé au cours de réactions ou en tant que réactif. Ainsi, dans le brevet US2400012 A, il est décrit un procédé d'obtention du para-cymène par déshydrogénation catalytique. Cette réaction engendre la formation d'un mélange composé de para-cymène et de para-menthane. Dans cette invention, le para-menthane formé est réutilisé à des fins de recyclage dans la réaction de déshydrogénation. Le para-menthane est utilisé dans une autre invention (US2302463 et US2302468) en réaction en phase liquide avec de l'oxygène. Il y est décrit une réaction visant à la conversion du para-menthane pour obtenir des alcools, aldéhydes, cétones et acides. Un procédé de sulfurisation des terpènes est décrit dans le brevet (US2537297). La sulfurisation a lieu sur les mélanges de terpènes dont le para-menthane peut faire partie. Dans le brevet US2S97372, il est décrit l'obtention de cyclopentane hydrocarbonés et de benzénoides hydrocarbonés par réaction du para-menthane et d'isocamphane en présence d'un catalyseur de type palladium. Ainsi, ces inventions relatant une présence du para-menthane dans des réactions chimiques de transformation, ils ne concernent pas les propriétés de solvatation du para-menthane vis-à-vis de composés d'intérêt naturels.

Le para-menthane a été proposé comme composant d'un mélange pour l'élimination d'encres (JP2005023196). L'utilisation de mélanges pour le nettoyage contenant entre autres du para-menthane a été décrite par la société Refining Oils dans plusieurs brevets (US20040102351, US20040238006 et US6872263). Les inventeurs ont alors découvert une formulation d'un agent de nettoyage comprenant un composé terpénique monocyclique saturé et un surfactant non ionique. L'utilisation de ce mélange se fait par injection dans une phase vapeur circulant dans les conduits et récipients à nettoyer. Dans un dernier brevet de Refining Oils (US20110056694), il est décrit une méthode pour éliminer les paraffines et bitumes dans des procédés de raffinage pétroliers. L'agent de nettoyage décrit est composé de para-menthane avec ou sans additif de type surfactant. Le spectre d'action de cet agent vise les composés apolaires non naturels de très haut poids moléculaire et fonctionne de plus dans un processus utilisant ce mélange à l'état de vapeur. Une formulation à base de terpènes proche des brevets précédemment cités est reportée dans le brevet EP 0781842 A2. Ce dernier brevet décrit un mélange de nettoyage non liquide composé de 90% à 96,5% d'un composé halogéné (n-propyl bromide), de 0 à 6,5% de composés terpéniques et de 3,5 à 5% de composés de bas point d'ébullition. Cet agent de nettoyage chimique est utilisé en phase vapeur à des températures autour de 70°C pour dissoudre des huiles minérales, des huiles de silicones ou métaux. Dans toutes ces inventions visant à décrire des procédés de nettoyage, il n'est pas fait état de l'utilisation du para-menthane pour l'extraction et la solubilisation de composés naturels obtenus à partir de matières naturelles végétales.

Ainsi, le para-menthane a été mentionné pour des applications chimiques ou comme composant de mélanges dans des procédés de nettoyage. Un besoin persiste toujours sur l'identification de solvants alternatifs se substituant à l'hexane, qui permettent de réaliser des extractions ou de solubiliser spécifiquement des composés naturels ciblés, tout en obtenant des rendements au moins identiques.

### [OBJET DE LA PRESENTE INVENTION]

La présente invention concerne un nouveau solvant d'extraction et/ou de solubilisation organique comprenant au moins 99 % de para-menthane, un procédé d'extraction mettant en œuvre ledit solvant, et les extraits issus dudit procédé.

La figure 1 montre la structure chimique du solvant selon l'invention (1-isopropyl-4-methylcyclohexane (para-menthane)).

Dans un mode préféré de réalisation, la présente invention vise à définir une nouvelle utilisation, du para-menthane, en tant que solvant d'extraction de matériel biologique végétal et/ou animal et/ou procaryote, et de manière préférée, en tant que solvant d'extraction à partir de matrices végétales d'origine naturelle ou solvant de solubilisation de composés naturels. En particulier, les inventeurs ont découvert que le para-menthane pouvait être mis en œuvre dans des procédés d'extraction et de solubilisation des principales catégories de composés biologiques naturels tels que les lipides, les antioxydants, les arômes et fragrances, les colorants.

Parmi les composés naturels d'intérêt visés, il existe la catégorie des molécules odorantes de type terpéniques, rentrant dans la composition d'huiles essentielles, des colorants de type caroténoïdes, chlorophylles ou autre composés colorants lipophiles et des acides gras. Le solvant selon l'invention peut être utilisé pour l'extraction d'ensemble de ces molécules sous forme d'extraits végétaux présents dans les matières végétales d'origine naturelle. Ainsi les extraits peuvent être des arômes naturels, des oléorésines, des huiles ou corps gras d'origine végétale.

De manière plus détaillée, les composés biologiques naturels comprennent : les acides gras, les lipides, les huiles, les composés saponifiables, les composés insaponifiables, les phytostérols, les phénols, les tocophérols, les tocotrienols, les vitamines E, les vitamines, les monoterpenes, les diterpènes, triterpènes et composés de type tarpénoïdes saturés ou insaturés, les composés aromatiques, les arômes et modificateurs de goût, les parfums, les fragrances, les composés odorants, les huiles essentielles, les sesquiterpènes, les composés phénoliques dont les acides, esters phénoliques et composés mono-phénoliques, les flavonoïdes, les tannins, les coumarines, les stilbénes, les lignanes, les sécoiridoïdes, les isoflavones, les acides phytiques, les alcools phénoliques, ainsi que les saponines, les antioxydants, les caroténoïdes, les chlorophylles, les xanthophylles, les curcuminoides, les bétalaines, les colorants, les pigments, les alcaloïdes, les phénylpropanoïdes, les biocides, les antimicrobiens, les protéines, les enzymes.

L'invention vise ainsi une nouvelle utilisation du para-menthane en tant que solvant d'extraction, ledit solvant étant obtenu à partir de matière première naturelle, dans le but de mettre en œuvre un procédé d'extraction plus respectueux de l'environnement, tout en permettant d'obtenir des rendements d'extraction équivalents à ceux obtenus avec l'hexane.

Le solvant selon l'invention peut ainsi être employé pour l'extraction et la solvatation de composés biologiques naturels d'intérêts présents en phase liquide ou contenus par exemple dans une matrice végétale d'origine naturelle.

Le para-menthane constitue un squelette de base des terpénoides composant les huiles essentielles ou autres composés odorants. Les inventeurs ont noté que le para-menthane pouvait être retrouvé à l'état de trace dans certaines plantes. Ainsi, l'une des caractéristiques recherchées d'un solvant est que ledit solvant doit être le plus possible inerte et ne pas être réactif en solution que ce soit durant le procédé d'extraction ou durant le stockage en cuve de celui-ci. Un solvant composé de molécules ne contenant pas de doubles liaisons telles que le solvant selon l'invention répond à cette contrainte.

En effet, l'homme du métier sait que les alcanes (tel que le para-menthane) possèdent une chaîne carbonée saturée très stable, avec des liaisons σ, qui est très difficile à casser sans avoir recours à des conditions extrêmes de température et de pression en présence de catalyseurs spécifiques. Il sait aussi que les alcènes sont moins stables et plus réactifs à cause de la présence d'insaturations : la présence d'une ou plusieurs double liaison π forme une double liaison plus réactive et moins stable que la liaison σ.

Le solvant d'extraction et/ou de solubilisation de matériel biologique végétal et/ou animal et/ou procaryote selon l'invention est caractérisé en ce qu'il comprend au moins 99 % de para-menthane. Dans un tel cas, les impuretés et composés initiateurs de réaction d'oxydation représentent au sein du solvant selon l'invention, moins de 1%.

Dans des modes d'extraction particuliers, il peut être nécessaire d'obtenir un extrait sous forme pure. L'élimination du solvant selon l'invention est alors requise pour obtenir des extraits exempts de solvant Ledit solvant peut être éliminé par différents procédés :
- par évaporation sous vide : dans ce mode de réalisation, l'injection d'éthanol ou d'eau au solvant selon l'invention permettra la formation d'un azéotrope et ainsi conduira à évaporation à température plus basse que le point d'ébullition dudit solvant seul ;
- par distillation moléculaire sous vide poussé.

De plus, le solvant selon l'invention étant composé d'une molécule inerte, il peut être facilement recyclé pour d'autres extractions sans qu'il y ait la formation de composés d'oxydation.

La présente invention concerne également un procédé d'extraction de composés actifs issus de matériaux biologiques notamment d'origine végétale en une seule phase. En effet, les inventeurs ont découvert que le solvant selon l'invention pouvait être mis en œuvre pour l'extraction et la solubilisation de composés thermosensibles. De manière surprenante, les rendements d'extraction sont proches de ceux obtenus à l'hexane, et le solvant selon l'invention permet une solubilisation infinie de composés purs miscibles. De plus, la mise en œuvre dudit solvant peut être réalisée à basse température.

Le procédé d'extraction selon l'invention est parfaitement compatible avec l'extraction de composés biologiques naturels tels que les composés phénoliques dont les phénols, les acides et esters phénoliques, les composés mono-phénoliques, les flavonoïdes, les tannins, les coumarines, les stilbènes, les lignanes, les sécoiridoïdes, les isoflavones, les acides phytiques, les alcools phénoliques, ainsi que les antioxydants, les caroténoïdes, les alcaloïdes, les phénylpropanoïdes, les monoterpenes, les diterpènes, triterpènes et composés de type terpénoïdes saturés ou insaturés, les sesquiterpènes, les arômes et modificateurs de goût, les huiles essentielles, les parfums, les fragrances, les composés odorants, les composés aromatiques, les biocides, les antimicrobiens, les protéines, les enzymes, les chlorophylles, les xanthophylles, les curcuminoides, les bétalaines, les colorants, les pigments, et les phytostérols, les acides gras, les tocophérols, les tocotrienols, les vitamines E, les vitamines, les lipides, les huiles, les composés saponifiables, les composés insaponifiables, comprenant les saponines.

Ce procédé d'extraction est caractérisé par une procédure comportant les étapes successives suivantes :
(a) mise en contact d'un matériel ou d'une matrice biologique végétal(e) et/ou animal(e) et/ou procaryote, broyé ou non, avec le solvant tel que décrit dans l'invention et/ou un mélange de ce solvant avec d'autres solvants, puis
(b) séparation du matériel ou de la matrice biologique végétal(e) et/ou animal(e) et/ou procaryote solide résiduelle,
(c) puis élimination partielle voir complète du solvant tel que décrit dans l'invention et/ou du mélange de ce solvant avec d'autres solvants.

De manière préférée, l'extraction sera effectuée à partir d'une matrice ou matériel végétal d'origine naturelle.

Dans un autre mode de réalisation, le solvant selon l'invention peut être utilisé pour solubiliser plus spécifiquement les composés lipophiles ou partiellement polaires dans des opérations d'extraction liquide / liquide.

Les paragraphes ci-après décrivent de manière nullement limitative, un exemple détaillé de mise en œuvre du procédé d'extraction selon l'invention.

L'extraction est réalisée à partir de matière première d'origine végétale provenant de différentes parties d'un végétal. Il peut s'agir de graines, de rhizomes, de racines, de tubercules, de tiges, de feuilles, de fleurs, d'écorces. Tout ou parties d'un végétal peuvent faire l'objet d'une extraction, seuls ou en mélange. Pour la préparation du végétal, il peut s'agir de toute opération unitaire visant à favoriser l'extraction, telles que le séchage, le broyage, l'aplatissage ou floconnage.

Pour la mise en œuvre dans des procédés d'extraction, le solvant selon l'invention peut être utilisé sous forme pure ou en mélange avec d'autres solvants qui y seraient miscibles. Les parties suivantes décrivent en détails, le procédé d'extraction faisant l'objet de la présente invention.

### Dans l'étape (a) :

Le solvant selon l'invention peut être utilisé dans des opérations conventionnelles d'extraction de matrices végétales d'origine naturelle, opérations telles que la macération (avec ou sans moyen d'agitation mécanique), la percolation, la lixiviation, ainsi que dans des procédés d'extraction assistée par des technologies innovantes tel que les ultrasons et micro-ondes.

La matière végétale sera de préférence sous une forme broyée afin de se présenter sous forme de fragment d'une granulométrie fine à moyenne de manière à faciliter l'étape d'extraction. La matière végétale fraîche aura avantageusement une teneur en eau de 40 à 90%, de préférence de 80 à 90%, en masse.

Le ratio solide végétal / liquide peut être compris entre 1/1 à 1/50 (en masse par volume) et de préférence à 1/20 en masse par volume. La température d'extraction peut être comprise entre 20°C et 150°C. Cependant pour les composés actifs d'origine naturelle, des températures modérées sont préférentiellement utilisées pour ne pas altérer la structure de ces composés. La température serait alors comprise avantageusement entre 40 et 60°C. La durée d'extraction est fonction du matériel végétal traité. La durée d'extraction peut être comprise entre 5 minutes et plusieurs heures. Les inventeurs n'ont pas noté de modification du solvant (changement d'aspect ou de propriétés) après 6 heures d'extraction. L'opération d'extraction peut avoir lieu à pression atmosphérique ainsi qu'à des pressions supérieures à la pression atmosphérique de manière à réaliser des extractions pressurisées. Ainsi le solvant selon l'invention peut être mis en œuvre dans des procédés ou opérations unitaires fonctionnant à pression égale ou supérieure à la pression atmosphérique.

### Dans l'étape (b) :

Les matières résiduelles solides sont éliminées du solvant par un procédé de séparation solide / liquide. Cette étape pourra être réalisée par filtration pour récupérer le solvant selon l'invention enrichi en composés d'intérêt extraits.

### Dans l'étape (c) :

Le solvant selon l'invention peut être éliminé de l'extrait solubilisé par différents procédés :
- par évaporation sous vide ; dans ce mode de réalisation, l'injection d'éthanol ou d'eau au solvant selon l'invention permettra la formation d'un azéotrope et ainsi conduira à l'évaporation à température plus basse que le point d'ébullition du solvant selon l'invention seul;
- par distillation moléculaire sous vide poussé.

A l'issue de cette étape, un extrait exempt de solvant est obtenu. De plus, le solvant selon l'invention récupéré à la suite de l'extraction reste stable car il ne comporte pas de double liaison. Son point d'ébullition élevé (168°C) implique également une volatilité bien inférieure à celle de l'hexane. Les pertes de solvants sont ainsi minimisées avec le solvant selon l'invention.

L'invention concerne également les extraits qui sont obtenus à l'issue de la mise en œuvre du procédé d'extraction avec le solvant selon l'invention.

Ces extraits pourront être utilisés pour la fabrication d'une composition nutraceutique, ou d'un produit diététique ou alimentaire, ou d'un complément nutritionnel, ou d'une composition pharmaceutique ou d'une composition cosmétique, destiné(e) à une administration selon le cas par voie orale ou parentérale, ou à une application par voie topique

Le solvant selon l'invention permet de solubiliser et d'extraire des composés présentant un caractère lipophile ou partiellement polaires. Ainsi le profil en composés extraits avec ce solvant peut différer de l'hexane ou de tout autre type de solvant apolaire. L'extrait obtenu avec le solvant selon l'invention présentera donc un profil unique. Ce dernier point pourra être illustré dans les exemples qui suivent.

Il est ainsi démontré que dans cette invention, l'intérêt de l'utilisation d'un nouveau solvant composé de para-menthane de pureté de 99%. L'extraction et la solubilisation de composés cibles naturels par le solvant selon l'invention sont effectives à des températures modérées. L'extraction de composés thermosensibles est alors rendue possible ; par exemple tels que les molécules odorantes de type terpéniques, rentrant dans la composition d'huiles essentielles, des colorants de type caroténoïdes, chlorophylles ou autre composés colorants lipophiles et des acides gras.

De plus, la structure chimique du solvant lui confère une stabilité réactionnelle et démontre des capacités d'extraction et de solubilisation similaires à l'hexane. Enfin, dû au point d'ébullition élevé du solvant selon l'invention, l'utilisation de celui-ci conduit à des pertes de solvant moins importantes qu'avec l'hexane. Ainsi, des exemples illustrant la mise en œuvre du solvant selon l'invention sont détaillés dans les sections suivantes.

### [MATERIELS ET METHODES]

Les extraits liquides obtenus sont directement analysés par chromatographie pour quantifier les composés bioactifs naturels présents en solution après extraction.

### Dosage du bêta-carotène

L'identification et la quantification du béta-carotène sont réalisées par Chromatographie Liquide Haute Performance (HPLC). Pour cela, un standard analytique a été utilisé (bêta-carotène, UV>98%, Extrasynthèse, référence 0303 S), et une droite de calibration a été établie. L'appareillage HPLC (Agilent 1100) est équipé avec un détecteur à barrettes de diiode. L'élution se fait en condition isocratique à 25°C, par la phase mobile composée du mélange acétonitrile/méthanol (avec 0.6% d'acétate d'ammonium)/dichlorométhane (77/20/3, v/v/v). Les solvants sont de qualité HPLC. La colonne utilisée est de type C18 (150 x 3,0 mm - 3 µm). La détection du bêta-carotène est réalisée à 464 nm.

### Dosage des acides gras dans l'huile de colza

L'identification et la quantification des acides gras dans l'huile de colza sont réalisées par Chromatographie en phase Gazeuse (GC). L'appareillage utilisé (GC 3800 VARIAN) est équipé d'un détecteur FID. La colonne utilisée est de type DB23 60 m x 0,25 mm avec un film d'épaisseur de 0,25 µm. La colonne est placée dans un four soumis à une température initiale de 120°c durant 5 min. La température est augmentée à 200°C (à 5°C/min) puis maintenue durant 1 min. Enfin, la température est augmentée à 230°C (à 2°/min) puis maintenue durant 15 min. Le gaz vecteur est de l'hélium à une pression constante de 30 psi.

L'injection de L'échantillon se fait à 250°C avec un split de 1 pour 50. La détection est réalisée au niveau d'un détecteur FID à 300°C. Les acides gras présents sont identifiés par comparaison des temps de rétention d'un mélange de standards d'acides gras (Supelco 37 component FAME mix, Sigma-Aldrich, ref. CMR47885). Les composés présents sont reportés en pourcentage relatif exprimé par rapport au total des composés détectés.

### Dosage des composés aromatiques de l'huile essentielle de basilic

L'identification des composés aromatiques contenus dans l'huile essentielle de basilic est réalisée par Chromatographie en phase Gazeuse (GC). L'appareillage utilisé (GC 7890 Agilent) est équipé d'un détecteur FID. La colonne utilisée est de type VF-MAX 30m x 0,25 mm avec un film d'épaisseur de 0,25 µm. La rampe de température suivante du four dans lequel est placé la colonne a été utilisée (tableau 1). Le gaz vecteur est de l'hélium à un débit constant de 1.1 ml/min.

**Tableau 1.**

| Température (°C) | Augmentation (°C/min) | Temps de maintien de température (min) | Temps total (min) |
|---|---|---|---|
| 60 | 0,0 | 1,0 | 1 |
| 240 | 3,0 | 5,0 | 66 |

L'injection de l'échanitillon se fait à 250°C avec un split de 1 pour 100. La détection est réalisée au niveau d'un détecteur FID à 250°C. Les composés présents sont reportés en pourcentage relatif exprimé par rapport au total des composés détectés.

Les exemples qui suivent permettent d'illustrer la mise en œuvre de la présente invention.

### [EXEMPLES]

### EXEMPLE 1 : Solubilité du bêta-carotène dans le solvant défini dans l'invention en comparaison à la solubilité du bêta-carotène dans l'hexane.

La solubilité du bêta-carotène est comparée dans deux solvants : dans l'hexane, d'une part, et dans le solvant selon l'invention, d'autre part. Pour chaque solvant évalué, 20 mg de bêta-carotène, de pureté supérieure ou égale à 97% sont mis en contact avec 1 mL de solvant. L'ensemble est placé dans un ballon sous constante rotation à une vitesse de 200 rpm à 25°C durant 1h. Le mélange est ensuite centrifugé pour faire précipiter le bêta-carotène qui n'a pas été solubilisé dans le solvant testé. La concentration en bêta-carotène dans la phase surnageante ainsi obtenue est déterminée par HPLC à une longueur d'onde de 464 mn. La concentration de bêta-carotène (exprimée en pourcentage massique) dans l'hexane est de 0,101% et la concentration de bêta-carotène dans le solvant selon l'invention est de 0,102%.

### EXEMPLE 2 : Solubilité du carvone dans le solvant défini dans l'invention, en comparaison à la solubilité du carvone dans l'hexane.

La solubilité du carvone est comparée dans deux solvants : dans l'hexane, d'une part, et dans le solvant selon l'invention, d'autre part Pour chaque solvant évalué, 1 mL de carvone, de pureté supérieure ou égale à 99%, est mis en solution avec 1 mL de solvant L'ensemble est placé dans un ballon sous constante rotation à une vitesse de 200 rpm à 25°C durant 1h. Après la phase d'agitation, le mélange carvone-hexane et carvone-solvant selon l'invention est comparé visuellement (tableau 3).

**Tableau 3.**

| | Solubilisation du carvone dans l'hexane | Solubilisation du carvone dans le solvant selon l'invention e |
|---|---|---|
| Examen visuel | complète | complète |

Le solvant selon l'invention permet une solubilisation totale du carvone à un ratio de 1/1 et 2/1 (volume/volume) : il n'a pas été noté de séparation de phases.

### EXEMPLE 3 : Solubilité du linalol dans le solvant défini dans l'invention en comparaison à la solubilité du linalol dans l'hexane.

La solubilité du linalol est comparée dans deux solvants : dans l'hexane, d'une part, et dans le solvant selon l'invention, d'autre part. Pour chaque solvant évalué, 1 mL de linalol, de pureté supérieure ou égale à 97%, est mis en solution avec 1 mL de solvant. L'ensemble est placé dans un ballon sous constante rotation à une vitesse de 200 rpm à 25°C durant 1n. Après la phase d'agitation, le mélange linalol-hexane et linalol- le solvant selon l'invention est comparé visuellement (tableau 4).

**Tableau 4.**

| | Solubilisation du linalol dans l'hexane | Solubilisation du linalol dans le solvant selon l'invention |
|---|---|---|
| Examen visuel | complète | complète |

Le solvant selon l'invention permet une solubilisation totale du linalol à un ratio de 1/1 et 2/1 (volume/volume) : il n'a pas été noté de séparation de phases.

### EXEMPLE 4 : Solubilité du limonène dans le solvant défini dans l'invention en comparaison à la solubilité du limonène dans l'hexane.

La solubilité du limonéne est comparée dans deux solvants : dans l'hexane, d'une part, et dans le solvant selon l'invention, d'autre part. Pour chaque solvant évalué, 1 mL de limonéne, de pureté supérieure ou égale à 93%, est mis en solution avec 1 mL de solvant. L'ensemble est placé dans un ballon sous constante rotation à une vitesse de 200 rpm à 25°C durant 1h. Après la phase d'agitation, le mélange limonène-hexane et limonène-solvant selon l'invention est comparé visuellement (tableau 5).

**Tableau 5.**

| | Solubilisation du limonéne dans l'hexane | Solubilisation du limonène dans le solvant selon l'invention |
|---|---|---|
| Examen visuel | complète | complète |

Le solvant selon l'invention permet une solubilisation totale du limonéne à un ratio de 1/1 et 2/1 (volume/volume) : il n'a pas été noté de séparation de phases.

### EXEMPLE 5 : Solubilité de l'estragol dans le solvant défini dans l'invention en comparaison à la solubilité de l'estragol dans l'hexane.

La solubilité de l'estragol est comparée dans deux solvants : dans l'hexane d'une part et dans le solvant selon l'invention d'autre part. Pour chaque solvant évalué, 1 mL d'estragol, de pureté supérieure ou égale à 98%, est mis en solution avec 1 mL de solvant. L'ensemble est placé dans un ballon sous constante rotation à une vitesse de 200 rpm à 25°C durant 1h. Après la phase d'agitation, le mélange estragol-hexane et estragol- solvant selon l'invention est comparé visuellement (tableau 6).

**Tableau 6.**

| | Solubilisation de l'estragol dans l'hexane | Solubilisation de l'estragol dans le solvant selon l'invention |
|---|---|---|
| Examen visuel | complète | complète |

Le solvant selon l'invention permet une solubilisation totale de l'estragol à un ratio de 1/1 et 2/1 (volume/volume) : il n'a pas été noté de séparation de phases.

### EXEMPLE 6 : Solubilité de l'huile de colza dans le solvant défini dans l'invention en comparaison à la solubilité de l'huile de colza dans l'hexane.

La solubilité de l'huile de colza est comparée dans deux solvants : dans l'hexane d'une part et dans le solvant selon l'invention d'autre part. Pour chaque solvant évalué, 1 mL d'huile de tournesol est mis en solution avec 1 mL de solvant. L'ensemble est placé dans un ballon sous constante rotation à une vitesse de 200 rpm à 25°C durant 1h. Après la phase d'agitation, le mélange huile de tournesol-hexane et huile de tournesol-solvant selon l'invention est comparé visuellement (tableau 7).

**Tableau 7.**

| | Solubilisation de l'huile de tournesol dans l'hexane | Solubilisation de l'huile de tournesol dans le solvant selon l'invention |
|---|---|---|
| Examen visuel | complète | complète |

Le solvant selon l'invention permet une solubilisation totale de l'huile de tournesol à un ratio de 1/1 et 2/1 (volume/volume) : il n'a pas été noté de séparation de phases.

### EXEMPLE 7 : Extraction d'huile de colza à partir de graines de colza selon l'invention en comparaison avec une méthode d'extraction utilisant l'hexane.

Les graines de colza (*Brassica napus* L,) contiennent de 35 à 45% d'huile. Le profil en acide gras de l'huile de colza industrielle est dominé par l'acide oléique (C18:1, environ 60%, g pour 100 g).

L'extraction est réalisée par macération de 2 g de graines de colza, préalablement broyées, dans 20 mL du solvant selon l'invention (rapport matériel végétal/solvant : 1/10). La macération est réalisée à 50°C, durant 6 h, dans un ballon sous constante rotation à 200 rpm. Les particules végétales solides sont séparées du solvant d'extraction par filtration. Pour obtenir des échantillons de volume identique et comparer les extraits, le filtrat est introduit dans une fiole puis le volume est complété à 20 mL avec le solvant d'origine.
La même procédure d'extraction peut être effectuée avec l'hexane (solvant de référence).

Le profil en acides gras de l'huile de colza, extraite selon ce procédé, est analysé par Chromatographie en Phase Gazeuse couplée à un Détecteur par Ionisation de Flamme, Les résultats sont présentés dans le tableau 8. Les proportions d'acides gras présents dans l'extrait sont indiquées en g pour 100 g.

**Tableau 8.**

| nature de l'acide gras | hexane | Solvant selon l'invention |
|---|---|---|
| acide palmitique (C16:0%) | 4,6 | 4,7 |
| acide stéarique (C8:0%) | 1,76 | 0 |
| acide oléique (C18:1%) | 58,19 | 58,97 |
| acide linoléique (C18:2%) | 19,23 | 19,79 |
| acide linolénique (C18:3%) | 7,16 | 6,6 |
| acide gadoléique (C20:1%) | 2,1 | 0 |

Pour les acides gras majoritaires, l'huile extraite par le solvant selon l'invention présente une proportion équivalente à celle extraite à l'hexane.

### EXEMPLE 8 : Extraction de composés odorante à partir de feuilles de basilic selon l'invention.

Les feuilles de basilic contiennent de l'huile essentielle qui se caractérisée par la présence de composés terpéniques majoritaires tels que le linalol, l'estragol, l'eugénol et le méthyl eugénol.

L'extraction est réalisée par macération de 2 g de feuilles de basilic, préalablement broyées, dans 20 mL du solvant selon l'invention (rapport matériel végétal/solvant : 1/10). La macération est réalisée à 50°C, durant 6 h, dans un ballon sous constante rotation à 200 rpm. Les particules végétales solides sont séparées du solvant d'extraction par filtration. Pour obtenir des échantillons de volume identique et comparer les extraits, le filtrat est introduit dans une fiole puis le volume est complété à 20 mL avec le solvant d'origine.

Le profil en composés terpéniques obtenu par analyse en Chromatographie en Phase Gazeuse couplée à un Détecteur par Ionisation de Flamme est présenté dans le tableau 9. Les proportions de composés terpéniques présente dans l'extrait sont indiquées en g pour 100 g.

**Tableau 9.**

| nature du composé | Solvant selon l'invention |
|---|---|
| limonène (%) | 0,70 |
| eucalyptol (%) | 0,97 |
| trans-b-ocimène (%) | 0,10 |
| camphre (%) | 0,33 |
| linalol (%) | 3,69 |
| bergamotène (%) | 0,10 |
| b-caryophyllene (%) | 0,19 |
| terpinen-4-ol (%) | 0,35 |
| estragol (%) | 7,25 |
| methyl eugenol (%) | 0,49 |
| tau-cadinol (%) | 0,90 |
| eugenol (%) | 0,14 |

Le solvant selon l'invention permet l'extraction de composés odorants présents dans les feuilles de basilic.

### EXEMPLE 9 : Simulation COSMO-RS et propriétés physiques du solvant

L'approche de simulation est réalisée avec le logiciel COSMO-RS (Figure 1 : σ-profil de l'hexane et du p-menthane). La molécule est immergée dans un conducteur parfait, ce qui génère une distribution de charge autour de la molécule.
La figure 2 montre le σ-profil de l'hexane et du p-menthane.

Cette distribution de charge est segmentée et réduite à un histogramme, nommé σ-profil. Le σ-profil permet de caractériser une molécule, dans son état d'énergie le plus stable. Les σ-profils de l'hexane et de *p*-menthane sont semblables. Leur σ-profil donne un double pic large centré en σ = 0, c'est-à-dire qu'ils ne possèdent pas de moments électrostatiques. Les deux pics résultent de la contribution des atomes d'hydrogènes (σ < 0) et de la contribution des carbones (σ > 0). Il n'y a pas de différence majeure entre le profil de l'hexane et celui du *p*-menthane, mise à part une petite différence de hauteur des pics, qui est dû à leur nombre de carbone et d'hydrogène respectifs,

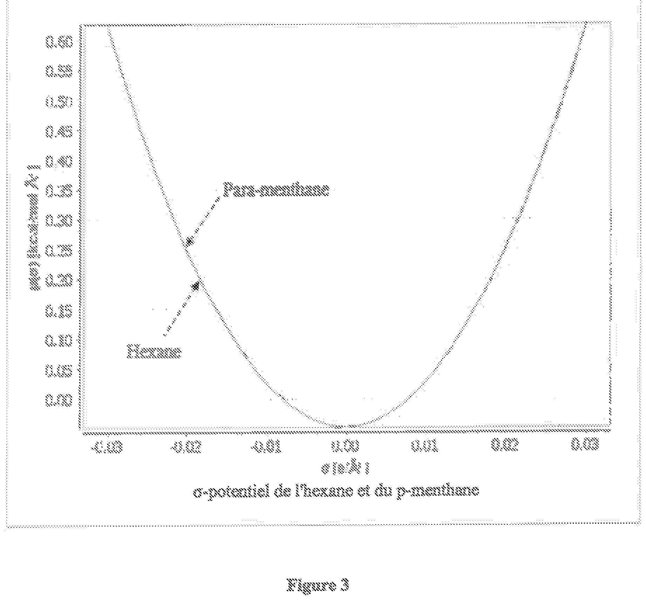

La figure 3 montre le σ-potentiel de l'hexane et du p-menthane.

Le calcul des σ-potentiels (figure 2) tient compte des interactions moléculaire deux à deux, ce qui permet de caractériser un solvant. Ici les σ-potentiels sont des paraboles centrées en σ = 0, cela exprime le fait que les alcanes n'ont aucune affinité pour les densités de charges positives ou négatives, traduisant leur caractère apolaire. La superposition des deux potentiels permet de calculer l'indice relatif de similarité entre les deux courbes. A 25°C, les deux molécules sont semblables à 95%. Le solvant selon l'invention paraît être un solvant prometteur pour remplacer l'hexane, qui est traditionnellement utilisé dans le domaine de l'extraction.

Enfin, les données physico-chimiques sont comparées dans le tableau 10 (ces données ont été obtenues par simulation à partir du logiciel ACD/lab).

**Tableau 10.**

| | *n*-Hexane | *p*-menthane |
|---|---|---|
| Formule brute | C₆H₁₄ | C₁₀H₂₀ |
| Densité | 0,7 ± 0,1 | 0,8 ± 0,1 |
| Température d'ébullition (°C) | 68,5 ± 3,0 | 168,2 ± 7,0 |
| Pression de vapeur à 25°C (mmHg) | 150,9 ± 0,1 | 2,2 ± 0,1 |
| Enthalpie de vaporisation (kJ / mol) | 28,9 | 38,8 ± 0,8 |
| Point éclair (°C) | -23,3 | 44,7 ± 11,7 |
| Indice de réfraction | 1,384 | 1,431 |
| Log P | 3,94 | 5,25 |
| Volume molaire (cm³) | 127,6 ± 3,0 | 178,6 ± 3,0 |
| Tension de surface (dyne/ cm) | 20,3 ± 3,0 | 23,9 ± 3,0 |

Le point éclair du solvant selon l'invention est plus élevé que celui de l'hexane ; ainsi le solvant selon l'invention présente une inflammabilité plus faible que celle de l'hexane. De plus, les deux solvants se distinguent principalement par leur température d'ébullition, pression de vapeur. L'hexane s'évapore à plus basse température que le solvant selon l'invention. La température d'ébullition du solvant selon l'invention est proche de celle composés terpéniques (168°C), et il est possible d'évaporer le solvant selon l'invention ;
- par évaporation sous vide : dans ce mode de réalisation, l'injection d'éthanol ou d'eau au solvant selon l'invention permettra la formation d'un azéotrope et ainsi conduira à l'evaporation à température plus basse que le point d'ébullition du solvant selon l'invention seul ;
- par distillation moléculaire sous vide poussé.

De plus, le solvant selon l'invention récupéré à la suite de l'extraction reste stable car il ne comporte pas de double liaison. Son point d'ébullition élevé (168°C) implique également une volatilité bien inférieure à celle de l'hexane. Les pertes de solvants sont ainsi minimisées avec le solvant selon l'invention.

## Revendications

1. Procédé d'extraction de composés biologiques naturels mettant en œuvre le solvant comprenant au moins 99 % de para-menthane.

2. Procédé d'extraction de composés biologiques naturels mettant en œuvre le solvant selon la revendication 1, lequel procédé comprend les étapes suivantes :
(a) mise en contact d'un matériel ou d'une matrice biologique végétal(e) et/ou animal(e) et/ou procaryote, broyé(e) ou non, avec ledit solvant et/ou un mélange de celui-ci avec d'autres solvants, puis
(b) séparation du solide résiduel du matériel ou de la matrice biologique végétal(e) et/ou animal(e) et/ou procaryote,
(c) puis élimination partielle ou complète dudit solvant et/ou du mélange de celui-ci avec d'autres solvants.

3. Procédé d'extraction de composés biologiques naturels selon la revendication 1 ou 2, dans lequel le matériel ou la matrice biologique mis(e) en contact avec le solvant est un matériel ou une matrice biologique végétal(e) d'origine naturelle.

4. Utilisation d'un solvant comprenant au moins 99 % de para-menthane pour extraire et/ou solubiliser des composés biologiques naturels.

5. Utilisation selon la revendication 4, dans laquelle les composés biologiques naturels sont choisis dans le groupe constitué par les acides gras, les lipides, les huiles, les composés saponifiables, les composés insaponifiables, les phytostérols, les phénols, les tocophérols, les tocotriénols, les formes de vitamine E, les vitamines, les monoterpènes, les diterpènes, triterpènes et composés de type terpénoïdes saturés ou insaturés, les composés aromatiques, les arômes et modificateurs de goût, les parfums, les fragrances, les composés odorants, les huiles essentielles, les sesquiterpènes, les composés phénoliques dont les acides, esters phénoliques et composés mono-phénoliques, les flavonoïdes, les tannins, les coumarines, les stilbènes, les lignanes, les sécoiridoïdes, les isoflavones, les acides phytiques, les alcools phénoliques, ainsi que les saponines, les antioxydants, les caroténoïdes, les chlorophylles, les xanthophylles, les curcuminoïdes, les bétalaïnes, les colorants, les pigments, les alcaloïdes, les phénylpropanoïdes, les biocides, les antimicrobiens, les protéines et les enzymes.

6. Utilisation selon l'une quelconque des revendications 1 à 3, dans laquelle les composés biologiques naturels sont choisis dans le groupe constitué par les acides gras, les lipides, les huiles, les composés saponifiables, les composés insaponifiables, les phytostérols, les phénols, les tocophérols, les tocotriénols, les formes de vitamine E, les vitamines, les monoterpènes, les diterpènes, triterpènes et composés de type terpénoïdes saturés ou insaturés, les composés aromatiques, les arômes et modificateurs de goût, les parfums, les fragrances, les composés odorants, les huiles essentielles, les sesquiterpènes, les composés phénoliques dont les acides, esters phénoliques et composés mono-phénoliques, les flavonoïdes, les tannins, les coumarines, les stilbènes, les lignanes, les sécoiridoïdes, les isoflavones, les acides phytiques, les alcools phénoliques, ainsi que les saponines, les antioxydants, les caroténoïdes, les chlorophylles, les xanthophylles, les curcuminoïdes, les bétalaïnes, les colorants, les pigments, les alcaloïdes, les phénylpropanoïdes, les biocides, les antimicrobiens, les protéines et les enzymes.

7. Extrait biologique naturel issu de matériel biologique végétal et/ou animal et/ou procaryote obtenu par la mise en œuvre du procédé selon l'une quelconque des revendications 1 à 3.

8. Utilisation d'un extrait selon la revendication 7 pour la fabrication d'une composition nutraceutique, ou d'un produit diététique ou alimentaire, ou d'un complément nutritionnel, ou d'une composition pharmaceutique ou d'une composition cosmétique, destiné(e) à une administration par voie orale ou parentérale, ou à une application par voie topique.

## Patentansprüche

1. Extraktionsverfahren für natürliche biologische Verbindungen, welches das Lösungsmittel umsetzt, das mindestens 99 % para-Menthan umfasst.

2. Extraktionsverfahren für natürliche biologische Verbindungen, welches das Lösungsmittel nach Anspruch 1 umsetzt, wobei das Verfahren die folgenden Schritte umfasst:
(a) Inkontaktbringen eines/einer pflanzlichen und/oder tierischen und/oder prokaryotischen biologischen Materials oder Matrix, zerkleinert oder nicht, mit dem Lösungsmittel und/oder einem Gemisch desselben mit anderen Lösungsmitteln, dann
(b) Trennen des Restfeststoffs des/der pflanzlichen und/oder tierischen und/oder prokaryotischen biologischen Materials oder Matrix,
(c) dann teilweises oder vollständiges Entfernen des Lösungsmittels und/oder des Gemischs desselben mit anderen Lösungsmitteln.

3. Extraktionsverfahren für natürliche biologische Verbindungen nach Anspruch 1 oder 2, wobei das biologische Material oder die biologische Matrix, das/die mit dem Lösungsmittel in Kontakt gebracht wird, ein/e biologische/s pflanzliche/s Material oder Matrix natürlichen Ursprungs ist.

4. Verwendung eines Lösungsmittels, das mindestens 99 % para-Menthan umfasst, zum Extrahieren und/oder Solubilisieren der natürlichen biologischen Verbindungen.

5. Verwendung nach Anspruch 4, wobei die natürlichen biologischen Verbindungen aus der Gruppe ausgewählt sind, die aus Fettsäuren, Lipiden, Ölen, verseifbaren Verbindungen, nicht verseifbaren Verbindungen, Phytosterolen, Phenolen, Tocopherolen, Tocotrienolen, Vitamin-E-Formen, Vitaminen, Monoterpenen, Diterpenen, Triterpenen und Verbindungen vom Typ gesättigter oder ungesättigter Terpenoide, aromatischen Verbindungen, Aromen und Geschmacksmodifikatoren, Duftstoffen, Düften, Riechstoffen, ätherischen Ölen, Sesquiterpenen, Phenolverbindungen, davon die Säuren, Phenolestern und Monophenolverbindungen, Flavonoiden, Tanninen, Cumarinen, Stilbenen, Lignanen, Secoiridoiden, Isoflavonen, Phytinsäuren, Phenolalkoholen sowie Saponinen, Antioxidantien, Carotinoiden, Chlorophyllen, Xanthophyllen, Curcuminoiden, Betalainen, Farbstoffen, Pigmenten, Alkaloiden, Phenylpropanoiden, Bioziden, antimikrobiellen Mitteln, Proteinen und Enzymen besteht.

6. Verwendung nach einem der Ansprüche 1 bis 3, wobei die natürlichen biologischen Verbindungen aus der Gruppe ausgewählt sind, die aus Fettsäuren, Lipiden, Ölen, verseifbaren Verbindungen, nicht verseifbaren Verbindungen, Phytosterolen, Phenolen, Tocopherolen, Tocotrienolen, Vitamin-E-Formen, Vitaminen, Monoterpenen, Diterpenen, Triterpenen und Verbindungen vom Typ gesättigter oder ungesättigter Terpenoide, aromatischen Verbindungen, Aromen und Geschmacksmodifikatoren, Duftstoffen, Düften, Riechstoffen, ätherischen Ölen, Sesquiterpenen, Phenolverbindungen, davon die Säuren, Phenolestern und Monophenolverbindungen, Flavonoiden, Tanninen, Cumarinen, Stilbenen, Lignanen, Secoiridoiden, Isoflavonen, Phytinsäuren, Phenolalkoholen sowie Saponinen, Antioxidantien, Carotinoiden, Chlorophyllen, Xanthophyllen, Curcuminoiden, Betalainen, Farbstoffen, Pigmenten, Alkaloiden, Phenylpropanoiden, Bioziden, antimikrobiellen Mitteln, Proteinen und Enzymen besteht.

7. Natürlicher biologischer Extrakt aus pflanzlichem und/oder tierischem und/oder prokaryotischem biologischem Material, erhalten durch das Durchführen des Verfahrens nach einem der Ansprüche 1 bis 3.

8. Verwendung eines Extrakts nach Anspruch 7 zur Herstellung einer nutrazeutischen Zusammensetzung oder eines diätetischen oder Nahrungsmittelprodukts oder einer Nahrungsergänzung oder einer pharmazeutischen Zusammensetzung oder einer kosmetischen Zusammensetzung, das/die zur oralen oder parenteralen Verabreichung oder einer topischen Anwendung bestimmt ist.

## Claims

1. Method for extracting natural biological compounds using a solvent comprising at least 99% para-menthane.

2. Method for extracting natural biological compounds using the solvent according to claim 1, which method comprises the following steps:
(a) bringing a crushed or uncrushed plant and/or animal and/or prokaryotic biological material or matrix into contact with said solvent and/or a mixture thereof with other solvents; then
(b) separating residual solid from the plant and/or animal and/or prokaryotic biological material or matrix;
(c) then partially or completely removing said solvent and/or the mixture thereof with other solvents.

3. Method for extracting natural biological compounds according to either claim 1 or claim 2, wherein the biological material or matrix brought into contact with the solvent is a plant biological material or matrix of natural origin.

4. Use of a solvent comprising at least 99% para-menthane for extracting and/or solubilising natural biological compounds.

5. Use according to claim 4, wherein the natural biological compounds are selected from the group consisting of fatty acids, lipids, oils, saponifiable compounds, unsaponifiable compounds, phytosterols, phenols, tocopherols, tocotrienols, vitamin E forms, vitamins, monoterpenes, saturated or unsaturated diterpenes, triterpenes and terpenoid compounds, aromatic compounds, flavourings and taste modifiers, perfumes, fragrances, odorous compounds, essential oils, sesquiterpenes, phenolic compounds including acids, phenolic esters and mono-phenolic compounds, flavonoids, tannins, coumarins, stilbenes, lignans, secoiridoids, isoflavones, phytic acids, phenolic alcohols, as well as saponins, antioxidants, carotenoids, chlorophylls, xanthophylls, curcuminoids, betalaines, dyes, pigments, alkaloids, phenylpropanoids, biocides, antimicrobials, proteins and enzymes.

6. Use according to any of claims 1 to 3, wherein the natural biological compounds are selected from the group consisting of fatty acids, lipids, oils, saponifiable compounds, unsaponifiable compounds, phytosterols, phenols, tocopherols, tocotrienols, vitamin E forms, vitamins, monoterpenes, saturated or unsaturated diterpenes, triterpenes and terpenoid compounds, aromatic compounds, flavourings and taste modifiers, perfumes, fragrances, odorous compounds, essential oils, sesquiterpenes, phenolic compounds including acids, phenolic esters and monophenol compounds, flavonoids, tannins, coumarins, stilbenes, lignans, secoiridoids, isoflavones, phytic acids, phenolic alcohols, as well as saponins, antioxidants, carotenoids, chlorophylls, xanthophylls, curcuminoids, betalaines, dyes, pigments, alkaloids, phenylpropanoids, biocides, antimicrobials, proteins and enzymes.

7. Natural biological extract derived from plant and/or animal and/or prokaryotic biological material obtained by the implementation of the method according to any of claims 1 to 3.

8. Use of an extract according to claim 7 for preparing a nutraceutical composition or a dietetic or food product, or a nutritional supplement, or a pharmaceutical composition or a cosmetic composition, intended for oral or parenteral administration or for topical application.
